# EUROPEAN PATENT SPECIFICATION

(11) **EP 3 902 498 B1**
(45) Date of publication and mention of the grant of the patent: **16.10.2024**
(21) Application number: 19906895.8
(22) Date of filing: 30.12.2019
(51) Int. Cl.: A61B 46/10, A61B 34/30

(54) **SURGICAL DRAPE FOR A ROBOTIC DEVICE**
CHIRURGISCHES ABDECKTUCH FÜR EINE ROBOTISCHE VORRICHTUNG
CHAMP OPÉRATOIRE POUR UN DISPOSITIF ROBOTISÉ

(30) Priority: 30.12.2018 US 201862786452 P
(43) Date of publication of application: 03.11.2021
(73) Proprietor: Momentis Surgical Ltd., 6037604 Or-Yehuda (IL)
(72) Inventor: LEVINSON, Yaron, 6037604 Or-Yehuda (IL); KONRAD, Michal, 6037604 Or-Yehuda (IL)
(74) Representative: Harrison IP Limited
(86) International application number: PCT/IL2019/051437
(87) International publication number: WO 2020/141517

(56) References cited:
- WO-A1-2011/154010
- WO-A1-2018/208898
- JP-A- 2009 153 859
- US-A1- 2006 161 137
- US-A1- 2006 161 137
- US-A1- 2015 257 841

## Description

### RELATED APPLICATIONS

This application claims the benefit of priority under 35 USC § 119(e) of U.S. Provisional Patent Application No. 62/786,452 filed December 30, 2018.

### FIELD AND BACKGROUND OF THE INVENTION

The present invention, in some embodiments thereof, relates to a surgical drape for covering a surgical device, and, more particularly, but not exclusively, to a surgical drape for covering a surgical robotic device onto which a distal extension can be assembled.

US Patent No. 8286637B2 discloses "A surgical radiological C-arm imaging unit drape configured to provide a sterile outer surface about an end portion of a C-arm imaging unit and method of providing a sterile surface about an end of a C-arm imaging unit is provided. The drape includes a flexible enclosure having an upper wall with a pair of sidewalls and a rear side extending downwardly from the upper wall. The upper wall and the side walls are extendible between expanded and collapsed positions. When in the expanded position, a pocket sized for receipt of the end portion of the imaging unit beneath the upper wall is formed. The upper wall has a sterile outer surface configured to face away from and overlie the end portion of the imaging unit to maintain sterility in a sterile zone above an operating table. When in the collapsed position, the sterile outer surface shielded from external contamination." (Abstract)

International Patent Application Publication No. WO 2018/208898 A1 disclosing a system for performing a medical procedure comprising a robotic introducer assembly including: a base unit, the base unit including a proximal base portion and a base extension extending from the proximal base portion; and a probe assembly operably and removably coupled to the base unit. The probe assembly includes an articulating probe. The base unit is constructed and arranged to control a movement of the articulating probe. The system further comprises a sterile barrier system positioned over at least a portion of the robotic introducer assembly, the sterile barrier system providing isolation between any non-sterile portion of the robotic introducer assembly and the sterile field or any sterile operators (e.g. to prevent exposure of any non-sterile portion to the sterile field and/or sterile operators). The sterile barrier system comprises a sterile frame having a first end coupled to a distal portion of the robotic introducer assembly and a second end coupled to a portion of the robotic introducer assembly proximal to a distal portion of the base unit. A sterile drape extends from the sterile frame and constructed and arranged for positioning about the base unit.

U.S. Patent Application Publication No. US 2006/161137 A1 disclosing a sterile drape, system, and method for draping portions of a telerobotic surgical system are provided. In one embodiment, a sterile drape includes an exterior surface adjacent to a sterile field for performing a surgical procedure, and an interior surface forming a cavity for receiving the non-sterile portion of a robotic surgical system, such as a manipulator. The drape further includes a fastener coupled to the exterior surface for securing the sterile drape to the non-sterile portion of the robotic surgical system while reducing the volume of the sterile drape. The drape allows for quick and simple installation and increases visualization of the patient by reducing the size of the drape with more form fitting features, while allowing for freedom of movement of the manipulator.

International Patent Application Publication No. WO 2011/154010 A1 disclosing an interventional drape and a kit for interventional procedures comprising the interventional drape. The interventional drape provided with at least one sterile pocket for procedural means and comprising a patient interventional drape with at least one fenestration, where a barrier drape is attached to said patient interventional drape and that said at least one pocket is provided in said barrier drape.

### SUMMARY OF THE INVENTION

According to an aspect of some embodiments of the invention there is provided a two-portion drape according to claim 1.

In some embodiments, the body of the first drape portion comprises at least one fixation element embedded or mounted onto the body and located between the accessible opening and the open end. In some embodiments, the fixation element comprises an elongated deformable element. In some embodiments, the body of the first drape portion is closed along the long edges of body. In some embodiments, the accessible opening is rectangular. In some embodiments, the second drape portion comprises a handling portion including valley folds each sized for insertion of at least a part of a user's hand. In some embodiments, the drape is formed of continuous or welded plastic or nylon sheets. In some embodiments, the drape is formed of one or more of thermoplastic polyurethane, ultra-high molecular weight polyethylene, low density polyethylene, and high density polyethylene. In some embodiments, the drape is at least partially transparent to allow visibility therethrough of at least a portion of a surgical device covered by the drape. In some embodiments, the first drape portion comprises a marker which extends at least around a perimeter of the accessible opening. In some embodiments, the marker comprises a colored lining. In some embodiments, the first drape portion comprises a pocket or fold in which the second drape portion is stored and can be pulled away from or inserted within. In some embodiments, the first drape portion and the second drape portion are unitarily formed. In some embodiments, the first drape portion and the second drape portion are formed as separate sheets. In some embodiments, one or both of the first drape portion and the second drape portion comprise attachment means including one or more of adhesives, hook and loop fasteners, velcro, magnets. In some embodiments, one or both of the first drape portion and the second drape portion comprise one or more markings for indicating a direction of dressing the drape portion over a surgical device. In some embodiments, the drape is formed of a material suitable for sterilization.

According to an aspect of some embodiments of the invention there is provided a method according to claim 15 for sterile covering of a surgical device including an access port and a surgical arm.

Unless otherwise defined, all technical and/or scientific terms used herein have the same meaning as commonly understood by one of ordinary skill in the art to which the invention pertains. Although methods and materials similar to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart of a method for covering a device comprising an attachable extension with a surgical drape, according to some embodiments;
FIG. 2 schematically illustrates deployment of a drape comprising a deformable element (e.g. a semi-rigid strip) and an opening for fitting onto an access port of a surgical device, according to some embodiments;
FIG. 3 is a flowchart of a method for covering a device comprising a modular extension with a surgical drape, according to some embodiments;
FIGs. 4A-B schematically illustrate a top face (4A) and a distal face (4B) of a device comprising a distal extension, when the device is covered by a sterile drape, according to some embodiments;
FIG. 5 is a flowchart of a method for covering a surgical device with a sterile drape comprising two separate sheets, according to some embodiments;
FIG. 6 is a flowchart of a method of replacing a distal device extension mid-surgery, while maintaining device sterility, according to some embodiments;
FIGs. 7A-B are schematic illustrations of bottom (7A) and top (7B) drape sheets for covering a surgical device comprising a distal extension, according to some embodiments;
FIG. 8 is a schematic drawing of a drape adhesive in the form of a pull-off tab, according to some embodiments;
FIG. 9 schematically illustrates a surgical drape comprising a fold-away portion, according to some embodiments;
FIGs. 10A-B are images of a bottom drape sheet deployed onto a motor unit of a surgical arm, according to some embodiments;
FIGs. 11A-B are images of a top drape sheet deployed onto a motor unit of a surgical arm, according to some embodiments;
FIG. 12 is an image of a deformable drape element in the form of a semi-rigid strip, according to some embodiments;
FIGs. 13A-C are images of an extended bottom drape (13A), a folded bottom drape (13B) and a folded top drape (13C), according to some embodiments;
FIGs. 14A-E schematically illustrate a drape sheet comprising a removable or movable portion, according to some embodiments;
FIGs. 15A-C are images of a drape sheet comprising an opening shaped and sized to expose an access port and an exit aperture of a motor unit, according to some embodiments;
FIG. 16 is a flowchart of a method of using a drape sheet including a detachable or movable portion, according to some embodiments;
FIGs. 17A-D schematically illustrate a drape for covering a surgical device and at least a portion of a base on which the surgical device is mounted, according to some embodiments; and
FIG. 18 is an image of drape covering a motor unit while exposing an access port and an exit aperture of the motor unit, according to some embodiments.

### DESCRIPTION OF SPECIFIC EMBODIMENTS OF THE INVENTION

The present invention, in some embodiments thereof, relates to a surgical drape for covering a surgical device, and, more particularly, but not exclusively, to a surgical drape for covering a surgical robotic device onto which a distal extension can be assembled.

A broad aspect of some embodiments of the invention relates to maintaining sterility of a surgical device which comprises an access port for attachment of a distal device extension. Some embodiments relate to limiting exposure of the access port in order to maintain sterility. Some embodiments relate to positioning of a surgical drape on the device in manner that enables modification (e.g. removal, attachment or replacement) of a distal extension such as a robotic arm (e.g. a surgical robotic arm), even during operation, by minimizing exposure of device areas that are not covered by the drape. Some embodiments relate to minimizing exposure of device portions that are uncovered by a surgical drape to a sterile environment, e.g. in an operating room.

An aspect of some embodiments of the invention relates to a surgical drape comprising at least one fixation, e.g. a deformable or flexible element, positioned to align or to maintain an alignment of the drape with respect to the device such that an opening defined in the drape is aligned with an access port of the device. In some embodiments, the fixation and the opening are respectively aligned, for example along a long axis of the drape. Optionally, the fixation and the opening are located at axially opposing portions of the drape. In some embodiments, the opening is defined in a drape portion which corresponds to a distal end of the surgical device, and the fixation is located in a drape portion which corresponds to a proximal end of the surgical device. Optionally, the opening and the fixation are aligned with respect to each other and with respect to the surgical device on which the drape is deployed. In some embodiments, a distance between the opening and fixation corresponds to a length of the surgical device. In an example of a drape for covering a motor unit of one or more surgical arm(s), the opening in the drape is located such that it is aligned with an access port through which the surgical arm(s) are attached, and the fixation is located such that it can be bent or otherwise fitted over a proximal end of the motor unit, e.g. bent over a corner or an edge connecting an upper face of the motor unit and a back face of the motor unit.

In some embodiments, the opening is shaped and/or sized according to the shape and/or size of the access port. According to one example, the opening is substantially rectangular. In some embodiments, a surface area of the opening matches that of the access port, or is optionally larger than that of the access port. In some embodiments, the opening comprises seams around its borders, optionally matching a position of the access port borders of the surgical device.

In some embodiments, the fixation element is deformable and/or flexible so that it can be fitted or bent to conform to a geometry of the surgical device or a portion thereof, e.g. bent over a corner of the surgical device. Optionally, the fixation element is non-rigid or semi-rigid. Optionally, the fixation element is plastically deformable. In some embodiments, the fixation element fixates the drape onto the device, setting the opening such that it fully overlaps the access port. In some embodiments, the drape overlies the device and encloses all device portions other than the access port and optionally an exit aperture from which the distal extension protrudes outwardly. In some embodiments, the fixation element is formed of a material which is rigid enough to fixate the drape onto the device and hold the drape in place, yet flexible enough to be bent by the user's fingers. In an example, a stiffness of the fixation element ranges between 1N/m to 10 N/m, such as 3 N/m, 5N/m, 7 N/m or intermediate, higher or smaller values.

In some embodiments, the fixation element tightens the drape over the device, stretching the opening across the access port. In some embodiments, the fixation element assists a user during deployment of the drape by indicating a desired position or orientation. In some embodiments, the fixation element is flexible enough to be folded over a device corner and/or to fit within or on a designated groove or marking formed on the device. In some embodiments, the fixation element acts as a frame for shaping at least a portion of the drape when deployed on the device. In some embodiments, the fixation element anchors the drape to the device to reduce or prevent movement of the drape, while maintaining access to the access port via the drape opening.

Optionally, the fixation element is shaped or in some embodiments re-shaped during use. Optionally, the fixation element is fitted onto a designated device location. In some examples, the fixation element is folded, stretched, twisted, and/or otherwise manipulated, optionally during use (e.g. during covering of the device) to conform to the shape of the device. The fixation element may be in the form of a rigid or semi-rigid strip, a clip, a string, a magnet, a hook and loop fastener (e.g. Velcro) and/or another element which can be flexed or bent to fit the device. Optionally, a fixation element in the form of a strip comprises a metallic element such as a metal wire, which in some embodiments is coated by a plastic lining. Optionally, the fixation element is embedded within a sheet of which the drape is formed. Alternatively, the fixation element is mounted onto the sheet.

An aspect of some embodiments of the invention relates to a surgical drape designed to provide for attachment, removal or replacement of a device extension while limiting exposure of device portions which are not directly associated with assembly of the extension, thereby reducing exposure of the device to the sterile operating environment and protecting the device from contamination (e.g. from contaminating fluids).

An aspect of some embodiments relates to a drape comprising a first portion including an opening shaped and sized to overlie the device access port, and a second portion comprising a cover that overlaps the opening, for example following loading of the device extension. In some embodiments, replacement or removal of the device extension (after initial loading of the extension) may be performed by removing, lifting, pushing aside and/or otherwise moving the cover away from the drape opening so as to expose the access port.

In some embodiments, the drape comprises two separate sheets. Optionally, the first sheet includes the opening and the second sheet includes the cover. Alternatively, a single sheet drape is provided, comprising a detachable cover portion or an integrated pocket or fold in which the cover is contained and can be pulled out from and compressed within.

In some embodiments, a drape is provided with a tear-off piece which can be removed or moved (e.g. folded or rolled away) to form the opening in the drape. Optionally, in use, the drape is dressed on the surgical device, and only close to or upon mounting or assembling of the distal extension through the access port, the tear-off piece is opened or removed, exposing the access port. In some embodiments, the drape is manufactured and packaged with the tear-off piece unitarily attached to the rest of the sheet. During use, a user (e.g. nurse, physician, technician) removes or moves the tear-off piece, such as by pulling the piece to detach along a line of dashed cuttings bordering the piece. Additionally or alternatively to a tear-off piece, a cover is provided, the cover being shaped and/or sized to at least partially overlap the opening through which the access port is exposed. During use, the user may detach, roll-up, push the cover aside and/or otherwise move the cover so as to expose the access port. A potential advantage of exposing the access port only close to or at the time of assembling the distal extension onto the surgical device may include reducing and optionally minimizing exposure of the device to the environment, potentially contributing to sterility maintenance.

A potential advantage of a removable or movable drape portion may include that the device portions covered by the drape portion, such as the access port, remain covered up until the step of attaching the device extension (such as the one or more surgical arms) via the access port.

In some embodiments, when the drape is deployed, a distal face of the drape is shaped to expose an exit aperture from which the distal device extension protrudes. In some embodiments, the drape is formed with opposing valley folds which when deployed define a cut-out portion between them which exposes the exit aperture. In some embodiments, the valley folds further serve as a handling portion, so that upon deployment a user places their hands within the valley folds and pulls the drape over the device.

In some embodiments, the drape comprises a flexible band which extends from a distal face of the drape and is long enough to be wrapped around the device extension at a location of the exit aperture. In some embodiments, the flexible band supports the device extension and assists in holding it place. In some embodiments, the flexible band covers any remaining exposed areas around the exit aperture, thereby potentially reducing a risk of contamination.

A potential advantage of a surgical drape configured for providing limited exposure of an access port and exit aperture of a distal extension may include maintaining sterility at the operating room environment, even when modifying (assembling, removing or replacing) the extension. Optionally, at least 85%, 95%, 99% percent or intermediate, higher or lower percentage of an external surface area of the device remain covered by the drape at all times. Optionally, no more than 15%, 20%, 5%, 30% or intermediate, higher or lower percentage of an external surface of the device is covered by the drape. In some cases, the device itself (e.g. a motor unit which actuates one or more surgical arms) is not sterile, and therefore needs to be covered for maintaining sterility of the surrounding operating room environment. Optionally, the one or more surgical arms attached to the motor unit are sterile, so that they can be used for performing operation in a patient. In an event that the arms are not sterile, the arms may also be covered by a protective sheath.

In some embodiments, the drape comprises one or more attachment means such as stickers, pull-off tabs, hook and loop type fasteners, dual lock fasteners, fasteners comprising an interlocking surface, and/or or other suitable means for attaching the drape onto the device and/or for attaching drape portions to each other. In some embodiments, a location of the one or more attachment means is selected to assist in positioning (for example align or orient the drape relative to the device) during deployment. In some embodiments, a location of the one or more attachment means is selected at areas prone to contact with contaminating factors and/or prone to entry of contaminating factors, for example one or more adhesives may be located around the borders of the opening formed in the drape to reduce a likelihood of contaminating factors entering between the drape and the device.

In some embodiments, a surgical drape is shaped and/or sized for covering, at least in part, a base on which the surgical device is mounted. In an example, the drape is large enough to cover a motor unit and a movable cart on which the motor unit is mounted. In some embodiments, the drape comprises a cut, tear-off portion and/or other movable or removable portion which provides for wrapping or otherwise placing the drape over the base, which, in some cases is of significantly larger dimensions than the surgical device itself. In some embodiments, the drape is placed over the base in a manner which provides for electrical connections (e.g. cables) and/or a handle of the base to protrude through the drape or an opening therethrough.

An aspect of some embodiments relates to an adhesive attachment in the form of a pull-off tab, which is structured for adherence of an adhesive strip without comprising sterility. In some embodiments, the pull-off tab comprises an elongate release paper that curls around from the internal side (the side that faces the device) of the drape to the external side of the drape. The internal end of the release paper is coupled to an adhesive strip so that when the external end is pulled on, the release paper is in turn pulled away from the adhesive strip, allowing the sterile user to adhere the inner side of the drape to the non-sterile device. A potential advantage of a structure in which no direct contact is formed with the adhesive strip may include maintaining sterility while still enabling attachment of the drape to the device.

A surgical device as referred to herein may include an activation unit of a robotic arm, such as a motor unit to which one or more surgical arms can be attached. Other device contemplated by this application may include, for example, surgical devices comprising one or more extensions, for example a driller and associated motor.

A device extension as referred to herein may include a surgical arm, for example a mechanical arm, optionally comprising one or more end-effecters for performing surgery (such as scissors, a grasper, an electrosurgery tip, and/or other surgical tools). In some embodiments, the device extension comprises a camera and/or other diagnostic means; a surgical working channel; microscopic surgery tools such as those used in infertility operations; and/or other surgical means which optionally extend from non-sterile machinery (such as a motor unit or control console) into a sterile environment.

As referred to herein, a "first drape sheet", "first drape portion", "base drape", or "base drape portion" may include a drape which is deployed first onto the surgical device and includes at least one opening, such as an accessible opening, which exposes an access port of the surgical device through, when the drape is deployed. Optionally, the accessible opening in the drape is shaped and sized to further expose an exit aperture of the surgical device, through which a device extension (e.g. one or more surgical arms) extends through. In some embodiments, the first drape includes at least one fixation element, e.g. a flexible or deformable strip, for fixating the drape onto the surgical device. In some embodiments, the first drape is sterilized, for example during manufacturing or later on, such as by EtO (ethylene oxide) sterilization process and/or other process suitable for reducing or eliminating infectious agents (e.g. bacteria).

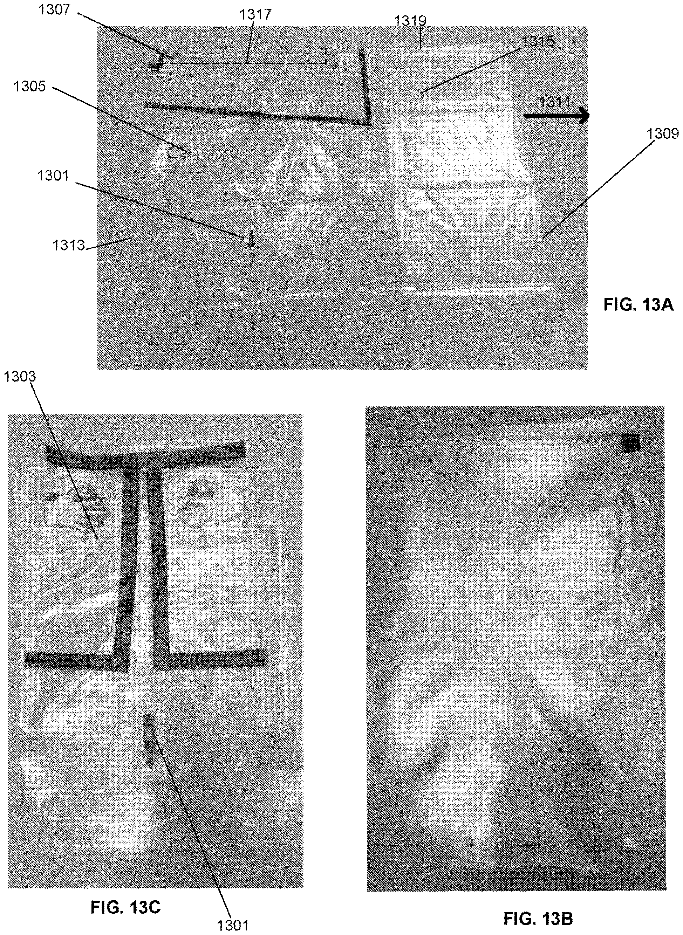

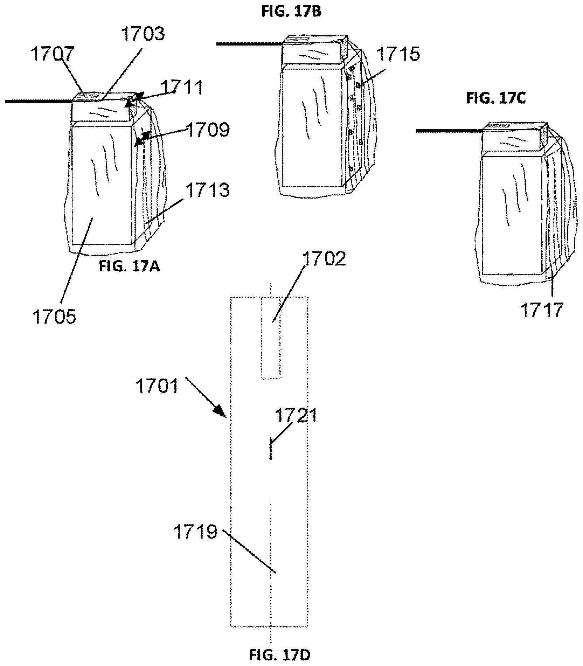

The first drape, when expanded from a folded, compact configuration (see for example FIG. 13B), forms a sleeve-like shape (see for example FIGs. 17A-C). The sleeve includes an open end, which provides for dressing the drape over the surgical device. In some embodiments, when dressing the drape over the surgical device, the direction of deployment is distal to proximal, from a front side of the device (which includes an exit aperture for the surgical arms) towards a rear side of the device. Then, in some embodiments, the margins of the drape are pulled or allowed to flow downwards, such as towards the ground.

In some embodiments, the first drape defines a longitudinal axis which substantially corresponds (or is parallel to) a longitudinal axis of the surgical device; a transverse (width) axis perpendicular to the longitudinal axis; and, in some embodiments, a height axis which extends, for example, from a top of the covered surgical device to a bottom end of the surgical device and/or to a bottom of a base on which the surgical device is positioned (as shown for examples in figures 17A-C).

In some embodiments, the accessible opening in the first drape is formed as two different openings. Optionally, a position and/or size of one of the openings corresponds to a location and/or size of the surgical device access port; and a position and/or size of the second opening corresponds to a location and/or size of the surgical device exit aperture.

In some embodiments, in a drape comprising a single accessible opening, when the drape is deployed, the accessible opening extends over a portion of a first face of the surgical device (e.g. a top face) to an adjacent, optionally perpendicular face of the surgical device (e.g. a distal face).

As referred to herein, a "second drape sheet", "second drape portion", "covering drape", or "covering drape portion" may include a drape which is deployed onto the surgical device following deployment of the first drape sheet or portion. In some embodiments, the second drape is shaped and sized to at least partially cover the accessible opening defined in the first drape. In some embodiments, the second drape covers a surface area of the accessible opening which corresponds to a location of the motor unit access port, and only partially covers (or, in some embodiments, does not cover) a surface area of the accessible opening which corresponds to a position of the motor unit exit aperture (i.e. from which one or more surgical arms extend). Optionally, the second drape fully covers the opening along one face of the covered surgical device (e.g. along a top face of the surgical device) and only partially covers the opening along an adjacent, optionally perpendicular face of the covered surgical device (e.g. a distal face).

In some embodiments, the second drape is formed as a sleeve. Optionally, the sleeve is sized with a larger cross section area than the first drape so as to be dressed over the first drape, optionally surrounding the first drape when deployed. Alternatively, the second drape is formed as a sheet and/or other shape. Optionally, the second drape is shaped and sized so that when it is dressed or disposed over the first drape it can be aligned with respect to the first drape and/or with respect to openings in the first drape and/or with respect to the surgical device.

In some embodiments, the second drape is sterilized, for example during manufacturing or later on, such as by EtO (ethylene oxide) sterilization process and/or other process suitable for reducing or eliminating infectious agents (e.g. bacteria).

A potential advantage of a two-part drape (or of two separate drapes) as compared to a single component drape may include minimizing exposure of a non-sterile area of the surgical device, preventing the need to replace the entire drape when attaching, removing and/or replacing the device extension, such as when attaching, removing and/or replacing one or more surgical arms on a motor unit covered by the drape(s).

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

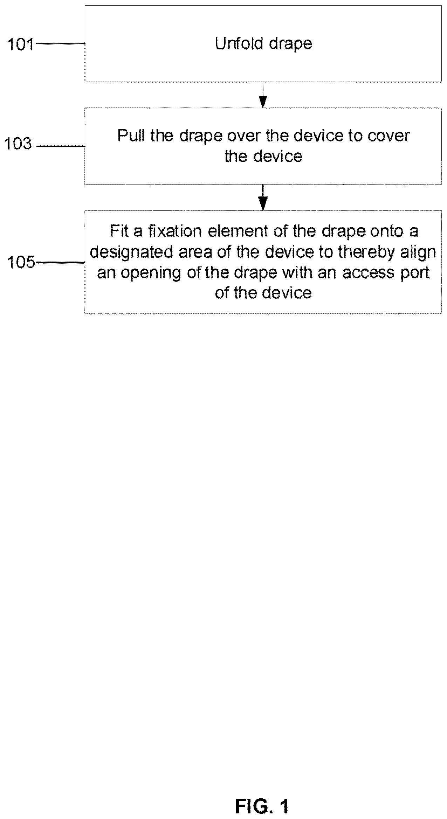

Referring now to the drawings, **Figure 1** is a flowchart of a method for covering a device comprising an attachable extension with a surgical drape, according to some embodiments.

For the purpose of maintaining sterility in an operating room, some surgical devices and commonly the patients themselves are covered by sterile covers. Protecting operating room equipment by sterile covers may in some cases reduce a risk of contamination, potentially reducing a risk of infection to the patient. In some embodiments, during preparation of the operating room, operating room personnel such as a physician, nurse, technician and/or other prepares the equipment by sterilizing it and/or placing protective drapes or other covers over the equipment or portions of it. In some embodiments, the surgical drape is placed in order to separate between sterile and non-sterile zones and/or components. In some embodiments, the surgical drape is placed in order to reduce contact of the device with potentially contaminating matter, such as biological fluids or the like.

In some embodiments, a surgical drape is unfolded (101) from a packed, folded state to an extended unfolded state in which the drape can be deployed onto a surgical device. In some embodiments, in the extended state the drape is spread out to a substantially flat form so as to overlie the surgical device. In some embodiments, the drape in its extended state is shaped and sized to cover the surgical device.

In an example, the surgical device comprises a motor unit of a robotic surgical arm, and the drape is constructed so that it covers the motor unit while still enabling one or more device extensions such as a robotic arm to protrude outwardly for performing the surgery. A potential advantage of such construction may include reducing the need to sterilize the motor unit itself. Another potential advantage may include providing for attaching and/or detaching the one or more robotic arms to or from the motor unit, while the motor unit remains substantially sterile, being covered by the drape. In some embodiments, the device extension (e.g. a robotic arm) is sterilized prior to use. Additionally or alternatively, the device extension is also covered or inserted within a sterile sheath. Optionally, the sheath is removed prior to introducing the extension into the patient's body.

In some embodiments, the drape is pulled over the device to cover it (103). Optionally, when deployed, the drape covers most of the exposed surface area of the device, for example covering at least the top and side surfaces of the device. In the example of a box shaped motor unit, the drape in its flattened extended state (before being placed on the motor unit) comprises a rectangular cross section such that when it is deployed onto to motor unit, it covers the top and side faces of the box-shaped motor unit, overlying the motor unit such that the free ends of the sheet are hanging over the edges of the motor unit. In some embodiments, the drape is sized to cover at least 80%, at least 85%, at least 90%, at least 95% or intermediate, higher or smaller percentage of the external surface area of the motor unit. In some embodiments, a user directs and aligns the drape during deployment, for example so that an opening of the drape overlaps the device access port.

In some embodiments, following initial placing of the drape onto the device (e.g. the motor unit), further adjustment of a location of the drape relative to the device may be required. In some embodiments, the drape is stretched over the device, centered with respect to the device, axially aligned with respect to the device and/or otherwise adjusted or repositioned for closely fitting the device (one could refer to this as being similar to setting a table cloth over the table). Therefore, in some embodiments, the drape comprises one or more elements for facilitating alignment of the drape with respect to the device and/or attachment of the drape onto the device to maintain the drape in a fixed position.

In an example, a fixation element in the form of a deformable semi-rigid strip is provided. In some embodiments, the semi-rigid strip is embedded in the drape sheet, or alternatively the strip is mounted onto the drape sheet. In an example, the semi-rigid strip comprises a coated flexible wire.

In some embodiments, following initial deployment of the drape onto the device, a user fits the fixation element onto the surgical device, for example pinches the semi-rigid strip onto a designated area of the device to thereby align or maintain an alignment of a different portion of the drape with respect to the device, for example an opening formed in the drape with an access port through which one or more device extensions can be attached (105). In some embodiments, the designated area of the device (e.g. a motor unit) onto which the semi-rigid strip is attached comprises a special shape, indication or marking for facilitating placing of the strip. For example, the location may be marked by an indentation, an elongate slot into which the strip can be inserted, a color marking, a bulge, a corner around which the strip can be folded, and/or other structural and/or visible features for indicating a location for attachment of the strip.

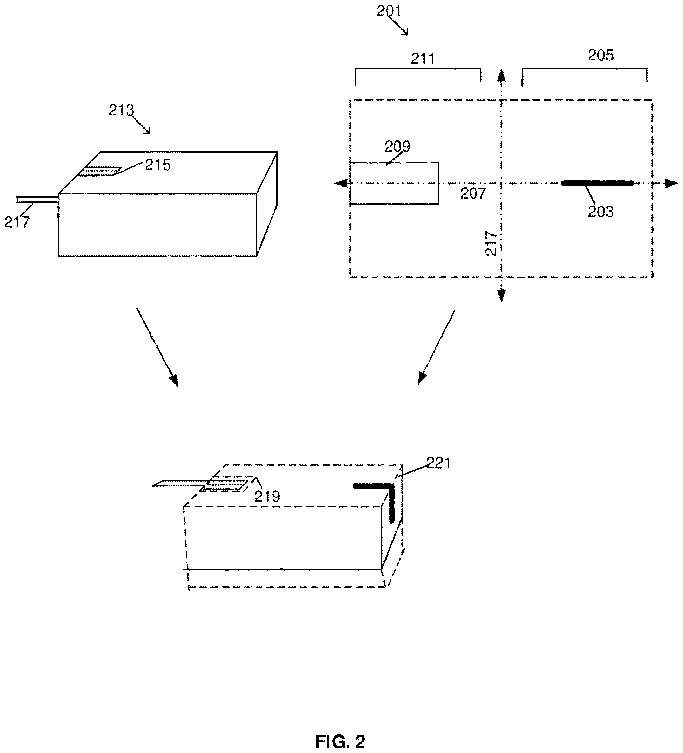

**Figure 2** schematically illustrates deployment of a drape comprising a deformable element (e.g. a semi-rigid strip) and an opening for fitting onto an access port of a surgical device, according to some embodiments.

In some embodiments, drape 201 is formed of a disposable, transparent, flexible, tearresistant material, such as plastic, nylon, and/or certain textile materials. In some embodiments, drape is formed of one or more of: thermoplastic polyurethane, ultra-high molecular weight polyethylene, low density polyethylene, and/or high density polyethylene. Optionally, the drape comprises welded or stitched sheets.

In some embodiments, drape 201 comprises a semi-rigid strip 203. Optionally, strip 203 is mounted or embedded within a proximal portion 205 of the drape. In some embodiments, strip 203 extends along a central long axis 207 of the drape, for example along 1/10, 1/8/, 1/5/, 1/3, 1/4 of a total length of axis 207. Strip 203 may extend from a most proximal point of axis 207, or, in some embodiments, from a point further along the axis.

In some embodiments, drape 201 is formed with an opening 209. In some embodiments, opening 209 is formed at a distal portion 211 of the drape. In some embodiments, opening 209 is shaped and/or sized so that when drape 201 is positioned to cover device 213, the opening overlaps an access port 215 of device 213 through which one or more device extensions 217 may be connected. In some embodiments, opening 209 is formed along central long axis 207. Optionally, opening 209 is rectangular. Other shapes of openings such as rounded, triangular, squared, or arbitrary shapes are also contemplated by this application.

In some embodiments, opening 209 and semi-rigid strip 203 are linearly aligned with respect to each other. Other embodiments may include different relative positions of the opening and the semi-rigid strip. Optionally, the opening and the semi-rigid strip are positioned across from each other, for example opposing each other on either ends of the drape sheet.

In some embodiments, when positioning drape 201 over device 213, the semi rigid strip 203 is pinched, folded and/or otherwise temporarily affixed to device 213. In the example shown herein, strip 203 is folded over a top proximally-facing corner of the device, thereby aligning opening 209 with the access port 215. In some embodiments, attaching the strip to the device (e.g. by folding over a corner, inserting into a designated slot, and/or other attachment methods) stretches the drape so that it closely fits onto at least to the top surface of the device. In some embodiments, attaching the strip to the device centers the drape with respect to the device, for example with respect to axis 217 extending along the wide dimension of the top surface of the device.

In some embodiments, the semi-rigid strip is configured to be plastically deformed during attachment. In some embodiments, the strip is deformed to conform to the shape of the external device housing, for example folded to conform to the angle of a corner 221 of the device. Optionally, deforming the strip modifies a planar arrangement of the drape. Optionally, the semi-rigid strip assists in maintaining the new planar arrangement assumed by the drape by supporting the drape portions, such as drape portions facing different directions following deformation of the strip.

In some embodiments, linings 219 of opening 209 of the drape include one or more deformable elements, such as semi-rigid strips. Optionally, the linings provide a supporting frame for the opening, which may assist in placing the opening at a position that overlaps with access port 215 of the device.

In some embodiments, opening 209 is shaped and/or positioned so that one or more device extensions 217 such as a surgical arm are allowed to extend from access port 215 in a distal direction.

Alternative embodiments may include a drape comprising an opening at a proximal portion of the drape, for example facing the proximal direction; such arrangement may be useful for a device in which an access port for receiving a device extension is located on a proximal face of the device (e.g. surgical arm insertion into the motor unit is performed from a proximal face of the unit). Other embodiments may include openings located at various locations, for example along side faces of the deployed drape sheet. Some embodiments may not comprise an opening. Optionally, a drape portion may be simply lifted or otherwise pushed aside to allow access to an access port.

In some embodiments, the drape comprises two openings, a first opening which aligns with the access port on a top face of the surgical device, and a second opening which aligns with an exit aperture of the surgical device through which the one or more surgical arms extend. Optionally, the exit aperture is positioned on a front (distal) facing face of the surgical device. Alternatively, a single opening of the drape is shaped and sized to expose both the access port of the surgical device and the exit aperture, optionally overlying adjacent (optionally perpendicular) faces of the surgical device.

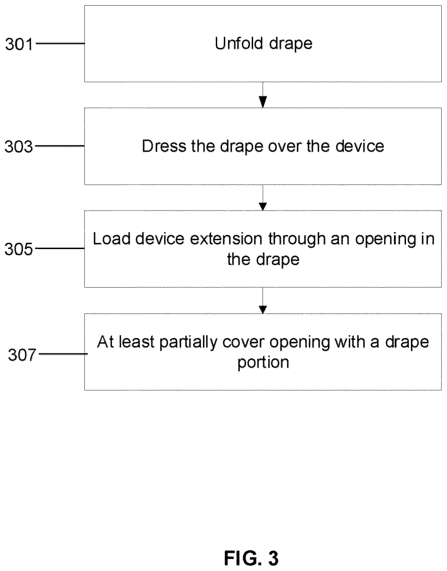

**Figure 3** is a flowchart of a method for covering a device comprising a modular extension with a surgical drape, according to some embodiments.

In some embodiments, the drape is unfolded from a compact, folded configuration (301). Then, the unfolded drape is dressed over the device (303). Optionally, deployment of the drape is assisted by one or more markings, openings, fasteners, plastically deformable elements and/or other components embedded within and/or mounted onto the drape. Additionally or alternatively, deployment of the drape is assisted by one or more markings, openings, protruding structures, indentations and/or other components of the device being covered.

In some embodiments, the drape is positioned over the device and orientated such that an opening formed in the drape is aligned with an access port of the device through which one or more device extensions may be attached. In some embodiments, once the opening overlaps the access port, the device extension is loaded onto the device (305). In an example, a surgical arm is loaded through one or more doors configured at the access port.

In some embodiments, optionally following loading of the surgical arm, the access port is at least partially covered with a drape portion (307). In some embodiments, the opening in the drape that was already fitted onto the device is covered by the drape portion, such that at least 95%, 90%, 85% or intermediate, higher or smaller percentage of the surface area of the opening is covered by the drape portion. In some embodiments, the drape or drape portion which covers, at least in part, the opening the defined in the initially deployed drape is shaped and sized to cover between 50%-80%, between 60%-90%, between 70%-95% or intermediate, higher or lower ranges of a surface area defined by the opening in the initially deployed drape. In some embodiments, the covering drape portion is shaped, sized and/or positioned such that it covers an access port of the surgical device, yet only partially covers or does not cover an exit aperture of the surgical device, through which the distal device extension (e.g. one or more surgical arms) may protrude.

In some embodiments, an integral drape sheet is provided, including a first portion in which the opening is formed and a second portion for further covering a device area exposed by the opening. Optionally, the second portion is seamed or welded to the first portion. Optionally, the second portion is attached to the first portion by a tear-off attachment.

In some embodiments, the second portion is packed in a folded state, and unfolded only when needing to cover the exposed device area, for example following loading of the surgical arm onto the device. Optionally, the folded portion is maintained in a designated pocket or fold created in the first portion. In some embodiments, the second portion can be pushed aside from the opening, for example so as to allow access to the access port (e.g. for removal or replacement of the device extension). Optionally, the pushed aside portion is grasped by clips, adhesive means and/or or other attachments to the device. Grasping the pushed aside portion to prevent it from contacting the surroundings may contribute to maintaining it sterile.

Additionally or alternatively, the second portion can be torn-off (and optionally disposed of) upon removal or replacement of the device extension.

Alternatively, the drape is comprised of two separate sheets, the first being formed with an opening, and the second sheet being shaped and sized to substantially cover the device area exposed by the opening. In some embodiments, the second sheet is removed (and optionally disposed of) upon removal or replacement of the device extension. Optionally, the first sheet remains in place on the device, and the second sheet is replaced by a new sheet.

A potential advantage of a drape comprising a movable or removable portion for placing over an access port and/or over an exit aperture from which the device extension extends may include providing for attachment, removal or replacement of the device extension, even during surgery, while reducing exposure of the device.

In some embodiments, the second drape comprises more than one portion, for example a first portion which covers a top face of a motor unit (such as when the motor unit is covered by the first drape) and a second portion which covers a front (i.e. distal facing) face of the motor unit (such as when the motor unit is covered by the first drape). Optionally, the plurality of portions which constitute the second drape at least partially overlap with each other.

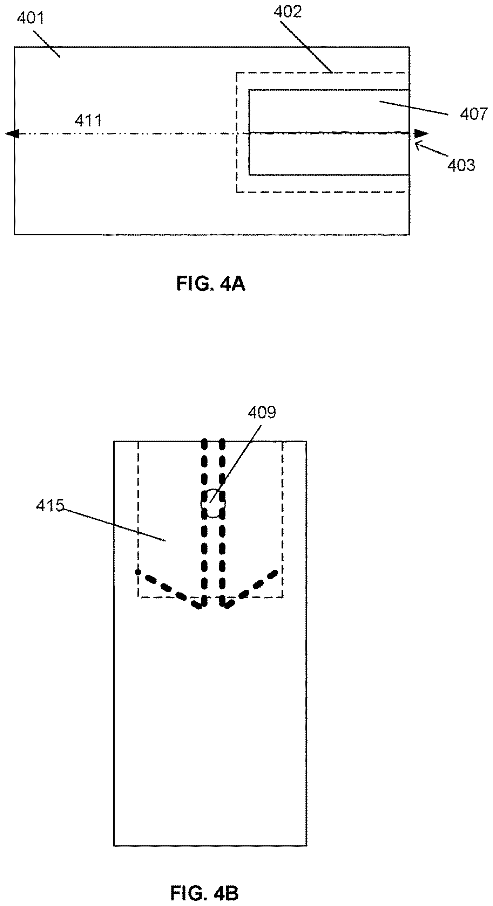

Figures 4A-B schematically illustrate a top face (4A) and a distal face (4B) of a device comprising a distal extension, when the device is covered by a sterile drape, according to some embodiments.

Drape 401 comprises an opening 402 ( formed as a cut-out portion) shaped and sized to expose an access port of the surgical device (in an example, a motor unit of a robotic arm). In some embodiments, the opening of the drape when deployed on the device extends from a top face onto a distal face, from which a device extension (not shown) extends distally. In some embodiments, the device extension is loaded via the access port, for example via one or more doors 407 located on the top face, such that it extends from a designated exit aperture 409 on the distal face of the device. In some embodiments, the loaded extension is linearly aligned along or parallel to a long axis 411 of the device. In some embodiments, doors 407 are also arranged along or parallel to long axis 411. Optionally, when doors 407 are closed, the device top face is flat, so that the doors do not interfere with drape deployment. A potential advantage of a flat surfaced device may include maximizing a contact area between the device surface and the drape, thereby minimizing entry of contaminating factors into the space between the device and the drape.

In some embodiments, a second drape portion (which may be integrally attached to drape 401, separable from drape 401 or in some embodiments is formed as an independent separate drape sheet) substantially covers opening 402 such that the exposed access port, exit aperture and surrounding areas are protected by the second drape portion. In some embodiments, the second drape portion includes opposing valley folds 415 in which the user's hands may be placed during deployment. When the second drape portion is deployed over the device, the valley folds define a curtain-like arrangement through which the device extension is allowed to protrude outwardly. In some embodiments, in addition to or instead of the valley folds, the drape comprises handling means such as a handle, strap, pulling cord, or other means which may facilitate deployment of the drape by a user.

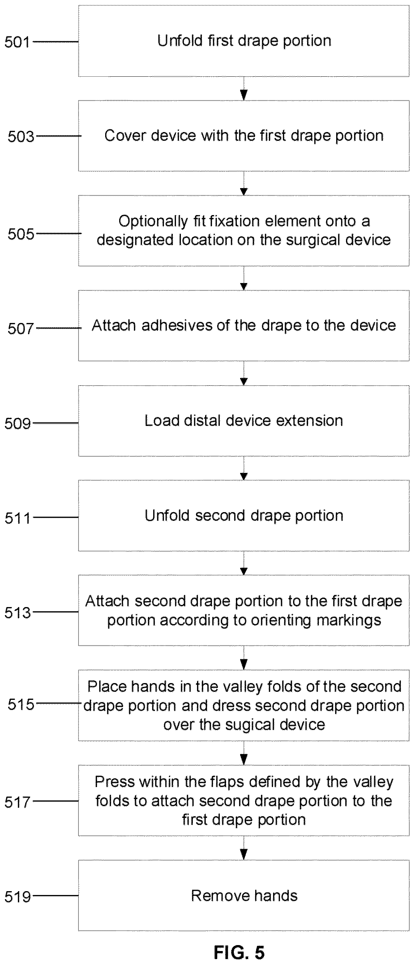

Figure 5 is a flowchart of a method for covering a surgical device with a sterile drape comprising two separate sheets, according to some embodiments.

In some embodiments, the drape is comprised of first and second (e.g. top and bottom) sheets, for deployment over the device before loading of the distal device extension and following loading of the distal device extension.

In some embodiments, a first drape portion (in an example, a first drape sheet) is unfolded from its compacted state and dressed over the device (501, 503). The drape is formed as a sleeve comprising an open bottom so that it can be dressed over the device and pulled to cover the device in the direction of the top surface of the device towards the bottom surface of the device. Optionally, one or more deformable elements such as a semi rigid strip are then manipulated by a user to attach the drape to the device (505), at pre-selected device locations or instead in an arbitrary fashion. In some embodiments, one or more attaching means such as adhesives are used for further coupling and/or aligning and/or sticking the drape to the device (507). Once the first drape portion is in place, an access port of the device remains exposed so as to allow for loading of a distal device extension through (509). Following loading of the distal device extension, a second drape portion (in an example, a second drape sheet) is unfolded from its compacted state (511). Optionally, the second drape portion is attached to first drape portion according to one or more orienting markings (513) (optionally, the markings include adhesives). Optionally, the marking are located at designated coupling locations between the first and second drape portions. In some embodiments, when folded, the second drape portion comprises valley folds for placement of the user's hands (515), which may assist in directing the user during deployment, for example by indicating a direction in which the second drape portion needs to pulled over the first drape portion. Optionally, the second drape portion is pulled from the proximal end towards the distal end of the surgical device, optionally along the long dimension of the device, until the valley folds are positioned extending along at least a portion of the distal face of the device. In some embodiments, the user then presses within the valley folds to attach the "flaps" defined by the folds onto the distal face of the device (517). Optionally, the flaps are positioned with respect to each other such that they allow the distal device extension to protrude between them. Then, the user removes their hands from the valley folds (519). At this stage, the deployed drape portions provide a sterile separation of the device from its environment.

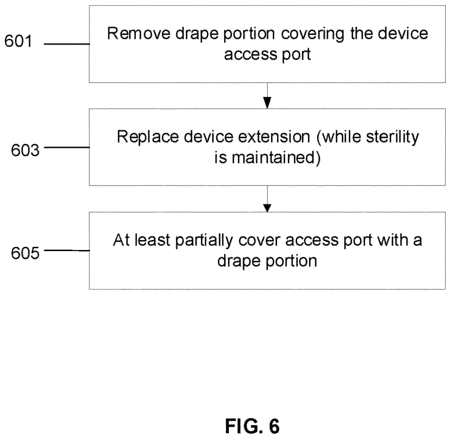

**Figure 6** is a flowchart of a method of replacing a distal device extension mid-surgery, while maintaining device sterility, according to some embodiments.

During surgery, it may be required to remove or replace the one or more device extensions, for example surgical arms. Such replacement may be performed in order to insert a surgical arm of a specific size or structure, to reduce or increase the number of arms being used, and/or to perform dedicated surgical actions such as drilling, stapling, suturing tissue, sharp dissection of tissue.

In some embodiments, a drape portion or separate drape sheet covering the exposed device access port and optionally an exit aperture of the distal extension is removed (601). Alternatively, the drape portion or separate drape sheet is pushed aside, folded away and/or otherwise moved from the device access port and/or from the exit aperture of the device extension. Optionally, the pushed aside portion is stored within a designated pocket formed in the drape or is otherwise held in a compacted or folded state so as to reduce its contact with the surroundings.

In some embodiments, removal of the drape portion exposes only a minimal surface area of the device, restricted for example only to the doors of the access port, so as to enable removal or replacement of the device extension (603). In some embodiments, only 5%, 10%, 15% or intermediate, higher or lower percentages of a total surface area of the device which was previously covered by the drape is exposed for the process of replacing or removing the device extension. A potential advantage of a drape designed for exposure of only a selected, minimal area of the surgical device may include a higher likelihood of maintaining sterility, for example as compared to a situation in which the full drape needs to be removed from the device to allow for removal or replacement of the device extension.

Replacement of the one or more device extensions, for example one or more surgical arms, may be performed by opening the access port doors, lifting the arm from the device, inserting a new arm through the open doors, and closing the doors to lock the arm in place.

In some embodiments, once the modification of the device extension(s) took place (e.g. removal, replacement, or addition of surgical arm(s)), the exposed device area is covered again by a drape portion. In some embodiments, a new drape sheet may be deployed onto the device. Additionally or alternatively, a drape portion that was previously stored away is placed back on the exposed device area (e.g. the access port) (605).

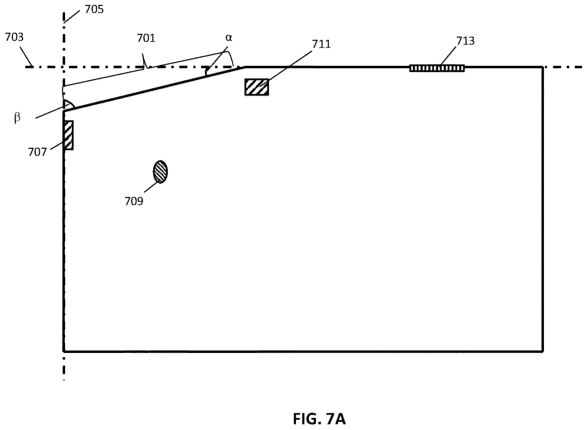

Figures 7A-B are schematic illustrations of bottom (7A) and top (7B) drape sheets for covering a surgical device comprising a distal extension, according to some embodiments.

The exemplary bottom drape sheet of figure 7A is shown at a cross section. An opening 701 (for example, a cut out portion of the drape sheet) is formed at the top distal corner of the drape. In some embodiments, opening 701 forms an angle α relative to a long axis 703 of the drape, ranging between, for example 1-30 degrees, such as 5 degrees, 10 degrees, 20 degrees or intermediate, higher or smaller angles. In some embodiments, an angle β, ranging between, for example, 60- 90 degrees, such as 70 degrees, 80 degrees, 85 degrees or intermediate, higher or smaller angles is formed relative to axis 705, extending along a height dimension of the bottom drape. Opening 701 is large enough to expose selected areas of the top and/or distal faces of the surgical device when deployed, for example an access port configured on the device top surface and an exit aperture of the distal extension on the device distal face.

In some embodiments, one or more adhesives such as a pull-off tab 707 are located along the distal face of the bottom drape, optionally directly beneath opening 701. In some embodiments, the pull-off tab is positioned to attach the bottom drape to the device at a location which is directly beneath the exit aperture of the extension. Another pull-off tab 711 may be positioned at the top face of the drape, adjacent opening 701. Positioning adhesives such as pull-off tabs adjacent borders of the drape opening may provide for ensuring adhesion of the drape at locations which may be prone to entry of contaminating factors. Additional adhesives such as hook and loop attachments 709 (for example, Velcro attachments) may be disposed at different locations along the drape. The bottom drape may include attachment means (such as adhesives) on an internal surface of the drape, which faces the device, and/or on an external surface of the drape, such as for attaching to the top drape.

In some embodiments, the bottom drape comprises a deformable element such as a semi-rigid strip 713 (for example as described hereinabove). Optionally, the bendable strip is located along long axis 703. Optionally, strip 713 is in proximity to a proximal side of the drape, so that upon deployment it can be bended over a top-proximal device corner, thereby potentially assisting in orienting and centering opening 701.

Exemplary dimensions of the bottom drape when extended may include: a length (measured along axis 703) of, for example, 2 m, 2.5 m, 3 m or intermediate, larger or smaller size, and a height (measured along axis 705) of, for example, 70 cm, 80 cm, 60 cm or intermediate, larger or smaller size.

In a compacted, folded state of the bottom drape (not shown herein), the dimensions may include: a length (measured along axis 703) of, for example, 250 cm, 200 cm, 300 cm or intermediate, larger or smaller size, and a height (measured along axis 705) of, for example, 70 cm, 80 cm, 60 cm or intermediate, larger or smaller size.

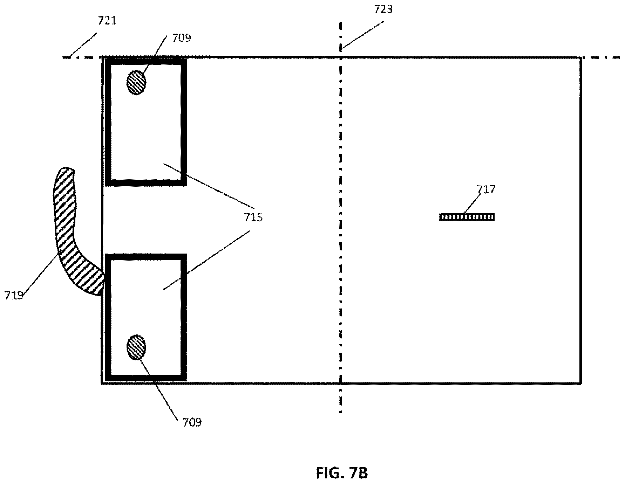

The exemplary top drape sheet of figure 7B is shown at an extended, spread out state. In some embodiments, the top drape comprises a set of opposing valley folds 715, configured for example along a distal face of the extended sheet. Optionally, a user places their hands in the valley folds during deployment onto the device, for pulling the top drape over the bottom drape. A potential advantage of the valley folds being designed for placement of the user's hands may include facilitating deployment of the top drape, reducing unnecessary contact between the user and the drape and contributing to directing and orienting the drape onto the device during deployment.

In some embodiments, adhesives such as hook and loop attachments 709 are positioned at an inner side of the valley folds, which faces the device distal face when the drape is deployed. In some embodiments, adhesives of the top drape are positioned to attach onto exposed surface areas of the device itself. Additionally or alternatively, adhesives of the top drape are positioned to attach onto an external side of the bottom drape.

In some embodiments, the top drape comprises one or more deformable elements such as a semi-rigid strip 717. Optionally, strip 717 is positioned at location which overlaps with strip 713 of the bottom drape upon deployment. This may assist in directing the top drape to a final deployed position.

In some embodiments, the top drape comprises a flexible band 719, for example formed of nylon, extending outwardly from a distal face of the drape sheet. In some embodiments, band 719 is long enough so that upon deployment of the top drape the band can be looped around an exit aperture of the device extension. By rolling band 719 around the exit aperture, and optionally around a shaft of the device extension, the band may contribute to: 1. Supporting and anchoring the device extension at the exit aperture and 2. Reducing an exposed surface area surrounding the exit aperture.

In some embodiments, dimensions of the top drape may include a length measured along axis 721 of, for example 400 cm, 500 cm, 750 cm or intermediate, larger or smaller size; and a width measured along axis 723 of, for example, 200 cm, 250 cm, 350 cm or intermediate, larger or smaller size.

Exemplary dimensions of the folded top drape sheet may include, for example, a length measured along axis 721 of, for example 200 cm, 213 cm, 250 cm or intermediate, larger or smaller size; and a width measured along axis 723 of, for example, 145 cm, 120 cm, 160 cm or intermediate, larger or smaller size.

In some embodiments, the bottom drape is large enough to cover the motor unit and further extend to cover a fixation arm which holds the motor unit to the operation table.

In some embodiments, the top drape is at least large enough to fully cover the opening of the bottom drape.

In some embodiments, the top and/or bottom drape sheets in their folded, compact states comprise indications (optionally including adhesives, folds, and/or color markings) for indicating the direction of unfolding and/or the direction of deployment over the device.

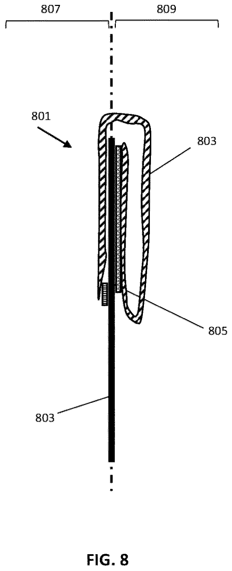

**Figure 8** is a schematic drawing of a drape adhesive in the form of a pull-off tab 801, according to some embodiments.

In some embodiments, the pull-off tab is structured to allow a user to pull the release paper 803 off to expose an adhesive strip 805 without comprising sterility. In use, a user, from the sterile side 807 (relative to drape sheet 808, that is- on the external side of the drape), pulls on release paper 803 which curls around to the non-sterile side 809 of the drape sheet. The release paper ends at the adhesive strip 807 so that due to the ringlet form of the release paper, the release paper can be pulled away from the adhesive strip without directly contacting the adhesive strip. When the adhesive strip is exposed, it can be pushed against a device surface (or against a different drape portion) to attach drape sheet 808.

Other attachment means may include stickers, clips, zippers, snaps, dual-lock fasteners (such as hook and loop fasteners), fasteners comprising flat interlocking fastener surfaces and/or other.

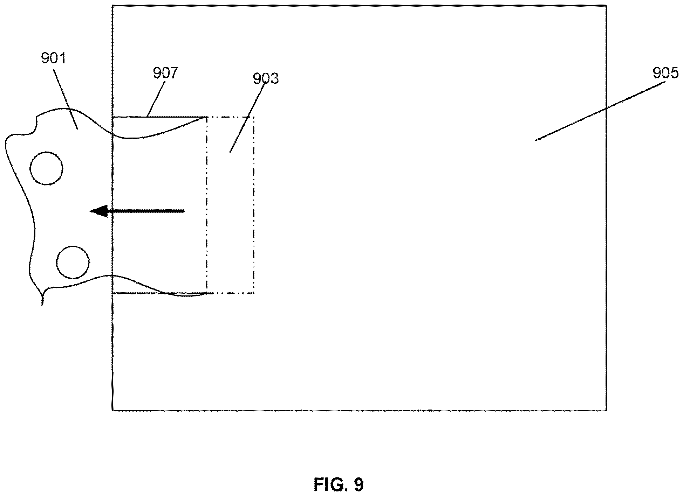

**Figure 9** schematically illustrates a surgical drape comprising a fold-away portion, according to some embodiments.

In some embodiments, a fold-away drape portion 901 is configured within a designated pocket or fold 903 formed in the drape sheet 905. Optionally, during use, drape sheet 905 is deployed onto the surgical device, such that an opening 907 of the drape sheet exposes an access port for attachment of a distal device extension and optionally an exit aperture of the distal device extension. During initial deployment, portion 901 may be stored away, and following loading of the device extension, portion 901 may be pulled or unfolded to cover exposed device areas. Optionally, portion 901 comprises one or more adhesives for attachment onto the device surface and/or onto selected locations of drape sheet 905.

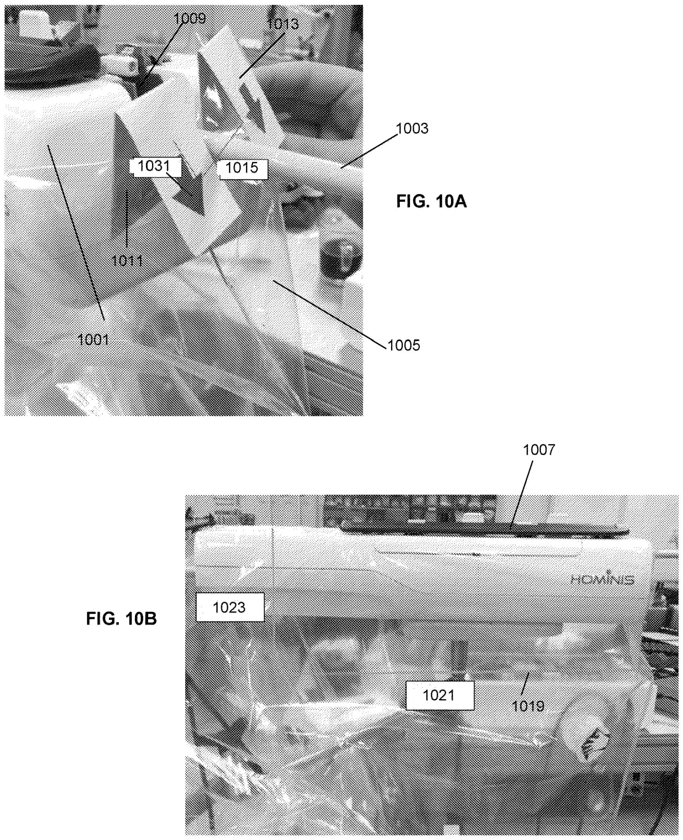

Figures 10A-B are images of a bottom drape sheet deployed onto a motor unit of a surgical arm, according to some embodiments.

Figure 10A is an image of a distal face of the motor unit 1001, from which a surgical arm 1003 extends outwardly in the distal direction. Figure 10B is a side view image of the motor unit. As can be observed, in some embodiments, the bottom drape sheet 1005 is disposed on the motor unit such that an opening formed in the drape exposes the access port including loading doors 1007 for attachment of the arm 1003, an further exposes an exit aperture 1009 from which the arm extends outwardly. In this example, a set of stickers 1011 are located adjacent exit aperture 1009 on opposite sides of the exit aperture, and can be attached to the motor unit surface upon pulling on the marked tabs 1013. In some embodiments, one or more arrows 1031 (or other directional markings) indicate a direction of pulling on the sticker.

In an exemplary embodiment, dimensions of a cut-out portion at the distal device face include a width 1015 of between 5 cm, 10 cm, or 15 cm or intermediate, larger or smaller size, for example so as to fit the drape directly from underneath and adjacent the exit aperture 1009.

In some embodiments, the bottom drape includes a colored line 1019 which marks the limits of the sterile zone. Exemplary dimensions of the sterile zone, as viewed from a side of the motor unit, may include a length 1021 of 300 cm, 250 cm, 400 cm or intermediate, larger or smaller size and a height 1023 of 15 cm, 20 cm or 25 cm or intermediate, larger or smaller size.

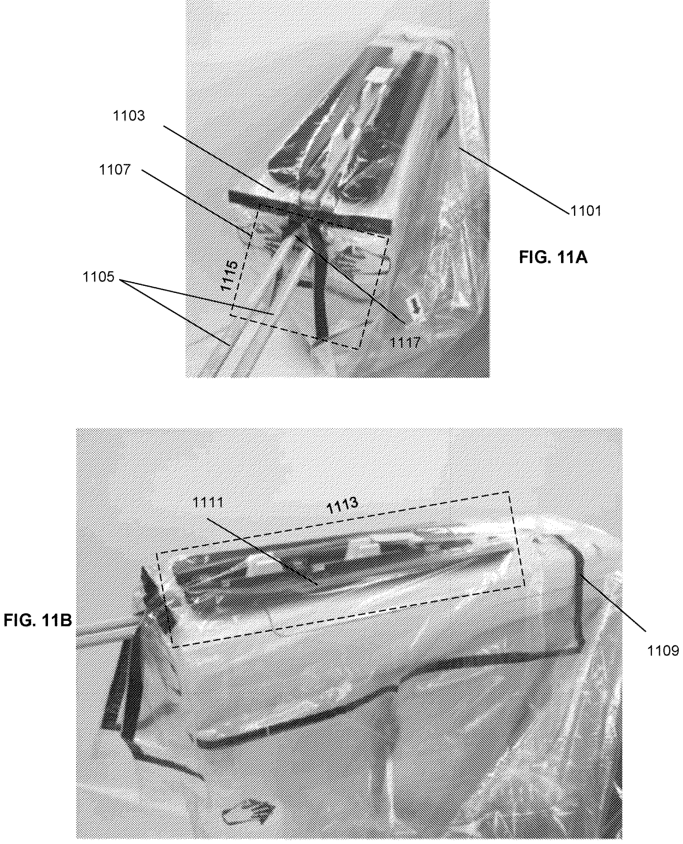

Figures 11A-B are images of a top drape sheet deployed onto a motor unit of a surgical arm, according to some embodiments. The front view of FIG. 11A shows the top drape 1101 covering the motor unit 1103 from which a pair of surgical arms 1105 extend distally. In some embodiments, the top drape comprises valley folds 1107 sized for placement of the user's hands during deployment over the motor unit. Optionally, at the final deployed position, valley folds 1107 extend along a distal face of the motor unit on opposing sides of an exit aperture from which the surgical arms extend.

Figure 11B is a side view of the covered motor unit. In some embodiments, the top drape includes a lining 1109 for indicating a sterile region.

In some embodiments, top drape 1101 comprises at least two portions: a first portion (schematically indicated at 1113, FIG. 11B) which is shaped and/or sized to cover at least the access port 1111 exposed by the opening in the bottom drape, and a second portion (schematically indicated at 1115, FIG. 11A) which is shaped and/or sized to extend along a front (distal) face of the motor while still exposing an exit aperture 1117 of the motor unit through which one or more of the surgical arms 1105 may protrude.

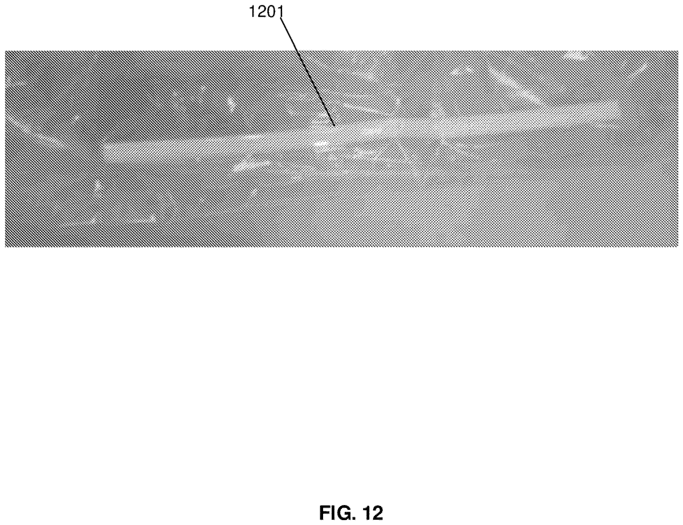

**Figure 12** is an image of a deformable drape element in the form of a semi-rigid strip 1201, according to some embodiments. In some embodiments, the semi-rigid strip comprises a wire coated by a plastic coating. Optionally, the strip is embedded within the drape material. Alternatively, the strip is mounted onto the drape.

Figures 13A-C are images of an extended bottom drape (when not deployed on the motor unit) (13A), a folded bottom drape (13B) and a folded top drape (13C), in accordance with some embodiments. In some embodiments, the top and/or bottom drapes include one or more markings such as arrows 1301 which are visible in the folded and/or extended positions and are located to assist directing the user during drape deployment.

In some embodiments, the valley folds 1303 for placement of the user's hands are accessible in the folded configuration of the drape.

In some embodiments, stickers 1305 or other markings which may assist in orientation during drape deployment are clearly visible in the folded and/or extended states of the drape.

In some embodiments, the top and/or bottom drapes include one or more adhesives 1307 for attaching the drape onto the motor device and/or for attaching the top and bottom drapes to each other.

As shown for example in Figure 13A, the bottom drape is sleeve shaped, and includes an open end (a direction of the open end indicated by 1309; the drape is shown folded, but when spread out it may extend longer in the direction indicated by 1311). A partially closed end 1313 of the sleeve is configured opposite the open end. body 1315 of the drape, extending between the open end and the partially closed end, is tubular and hollow.

As referred to herein, a "partially closed" end may include a side or an edge of the drape sleeve which is sealed along a portion of its length and open along another portion of its length. Optionally, the majority of the length is sealed. In some embodiments, in use, the partially closed end of the deployed drape overlies a distal face of the surgical device such that the exit aperture remains exposed but the rest of the distal face is covered.

An accessible opening 1317 (indicated by the dashed lines) is defined in the sleeve, extending from the partially close end 1313 to a side edge 1319 of the tubular body 1315 of the drape. The accessible opening is defined by a cut-out in the drape. In some embodiments, the accessible opening is rectangular. Optionally, as shown for example in this photo, the accessible opening comprises a stepped profile. In some embodiments, in use, a portion of the accessible opening (such as the portion extending along the close end of the sleeve) overlies an exit aperture of the surgical device (such as an exit aperture of a motor unit through which one or more surgical arms extend); and a different portion of the accessible opening (such as the portion extending along a segment of edge 1319) overlies the access port of the surgical device.

It is noted that the accessible opening may be rectangular, triangular, circular, arbitrary shaped, and/or having other shapes or profiles.

A potential advantage of a sleeve shaped drape may include reducing or avoiding the need to tie together or otherwise attach loose ends of a drape, such as a single layer flat sheet drape, when the drape is deployed to cover the surgical device. In some embodiments referred to herein, the surgical device is narrow and elongated and therefore deployment of a drape over the surgical device may be facilitated by use of a sleeve shaped drape. Another potential advantage of a sleeve shaped drape is that since no or less free ends of the sleeve need to be tied or otherwise attached to each other, sterile coverage is improved, and less exposure of the device to the environment (or vice versa) is provided.

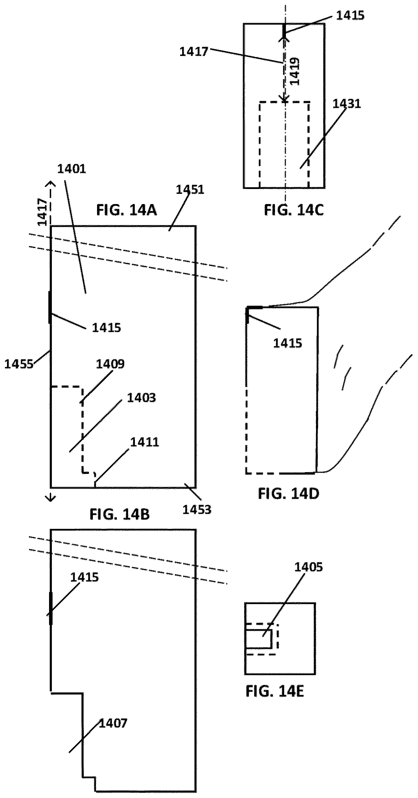

Figures 14A-E schematically illustrate a drape sheet including a removable portion which forms the opening in the sheet when removed, according to some embodiments.

In some embodiments, a drape sheet 1401 comprises a removable or movable portion 1403, which when removed (or moved) defines the opening 1407 in the drape sleeve through which the access port 1431 (see Figure 14C) of the surgical device is exposed. Optionally, portion 1403 is attached to the drape by a tear-off coupling, so that for example by pulling on the portion, it can be separated from the sleeve. Additionally or alternatively, the portion is removably attached to the sheet, e.g. by adhesives, e.g. via sticky tape, glue, and/or others.

Figure 14A schematically illustrates, from a side view, drape 1401 folded along the drape long axis while portion 1403 is still attached to the drape. Seams of the removable/movable portion 1403 are indicated by the dashed line. In Figure 14B, in accordance with some embodiments, portion 1403 is removed, thereby forming the opening through which a motor unit access port may be exposed. Figure 14C shows a top view of the drape sheet when deployed onto the motor unit, in accordance with some embodiments. Figure 14D shows a side view of the drape sheet when deployed onto the motor unit, in accordance with some embodiments. Figure 14E shows a front view of the drape sheet when deployed onto the motor unit, in accordance with some embodiments. Optionally, when portion 1403 is removed, an exit aperture 1405 of the motor unit, for example through which the one or more surgical arms extend distally, is exposed.

In some embodiments, portion 1403 (and therefore opening 1407 which is formed upon removal or moving of portion 1403) is shaped and sized to match a shape and/or size of the motor unit access port and exit aperture. Optionally, portion 1403 fittingly overlaps the access port and exit aperture so that it does not extend beyond the borderlines of the access port and exit aperture, exposing only those and no other portions of the motor unit when removed. Optionally, a surface area of portion 1403, corresponds to a summed surface area of the access port and exit aperture of the motor unit. Optionally, a surface area of portion 1403 is similar to the summed surface area of the access port and exit aperture. In the shown example, portion 1403 is shaped to have a stepped profile, defining for example two steps. Optionally, the majority of the opening constituted by an upper step 1409 is configured to expose the motor unit access port; and the minority of the opening constituted by a lower step 1411 is configured to extend directly below the exit aperture of the motor unit, when the drape is deployed on the motor unit (e.g. as shown in figure 14E).

Alternatively, portion 1403 is shaped and/or sized to be a larger than the access port and exit aperture. For example, portion 1403 may be formed with a rectangular side profile, where, for example, a lower edge of the rectangle extends below the exit aperture when the drape is deployed on the motor unit.

In some embodiments, as shown for example in figure 14C, portion 1403 defines an opening through which an access port of the surgical device is exposed, and a fixation 1415 for fitting the drape onto a proximal end of the surgical device (in an example- a deformable strip), are located at a distance 1419 of, for example, 10-20 cm from each other, such as 14 cm, 16 cm, 18 cm or intermediate, longer or shorter distance from each other as measured for example from a proximal end of portion 1403 to a distal end of fixation 1415. Optionally, portion 1403 and fixation 1415 are located opposite each other along an axis, in some embodiments being a long axis 1417 of the drape. Optionally, portion 1403 and fixation 1415 are centralized with respect to the long axis 1417. In some embodiments, distance 1419 corresponds to a distance between the access port of the surgical device (e.g. a motor unit) and the proximal end of the surgical device, e.g. a proximal face of the motor unit.

It is noted that in some embodiments the drape includes more than one fixation element for affixing the drape onto the motor unit, for example 2, 5, 6, 10 fixation elements or intermediate, larger or smaller number. Optionally, the different fixation elements are positioned on the drape in a positions corresponding with a motor unit edges or corners.

In some embodiments, as shown for example in figure 14A, the drape, for example when spread out (and in a non-deployed state), comprises an elongated hollow structure, optionally, a tubular sleeve shape. In some embodiments, the sleeve defines an open end 1451 and an opposing partially closed end 1453. In some embodiments, the open end allows for pulling the walls of the sleeve away from each other, so as to dress the sleeve on the surgical device.

In some embodiments, the opening defined at portion 1403 extends from an edge of the partially closed end 1453 to an adjacent edge 1455 of the tubular sleeve, which may be substantially perpendicular to the closed end 1453 at the spread out configuration.

In some embodiments, the drape long axis 1417 extends along edge 1455. In some embodiments, the opening defined at 1403 and the fixation 1415 are configured along the long axis.

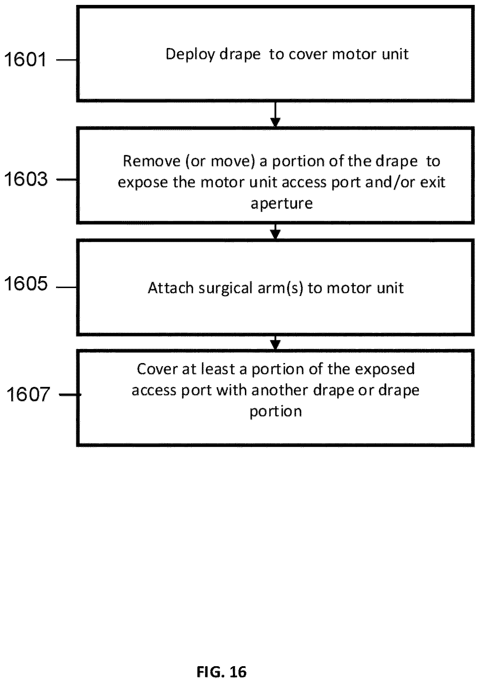

In some embodiments, during use, as described for example in the flowchart of **Figure 16**, after the drape had been deployed to cover the motor unit (1601), a user (e.g. nurse, physician and/or other surgical room staff), detaches the removable drape portion, thereby exposing the motor unit access port and/or exit aperture (1603). Additionally or alternatively, the user moves the drape portion, e.g. by rolling it sideways or upwards, pushing the portion aside, and/or otherwise exposes the motor unit access port. In some embodiments, removal and/or moving of the drape portion takes place only close to (e.g. right before) the time of attaching the surgical arm(s) to the motor unit, for example, no more than 5 minutes, 2 minutes, 30 seconds, 10 seconds or intermediate, longer or shorter time periods before attaching the surgical arm(s) to the motor unit. A potential advantage of exposing the motor unit access port by removing (or moving) the drape portion only at the time of attaching the surgical arm(s) to the motor unit may include minimizing exposure of non-sterile portions of the surgical device to the sterile environment surgical room environment. Another potential advantage may include protecting, for as long as possible, the motor unit (or portions thereof) from possible contamination. At 1605, the surgical arm(s) are attached to the motor unit, e.g. by mounting the arms via the exposed access port of the motor unit. At 1607, once the arms are in place, at least a portion of the access port is covered again, for example by a second drape sheet, by a different portion of the initially used drape sheet, and/or by the same portion that was moved or rolled aside to expose the access port. Optionally, at the end of the draping process, only the exit aperture remains uncovered by a drape so that the surgical arm(s) are allowed to extend therethrough.

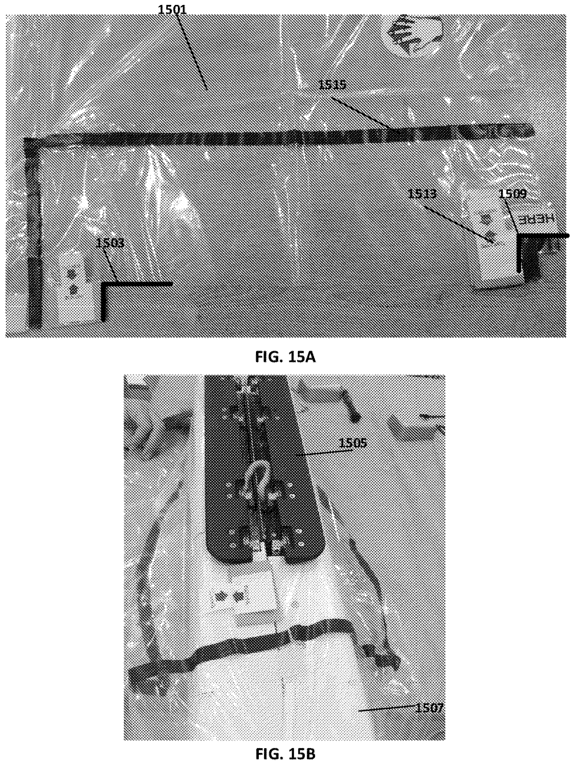

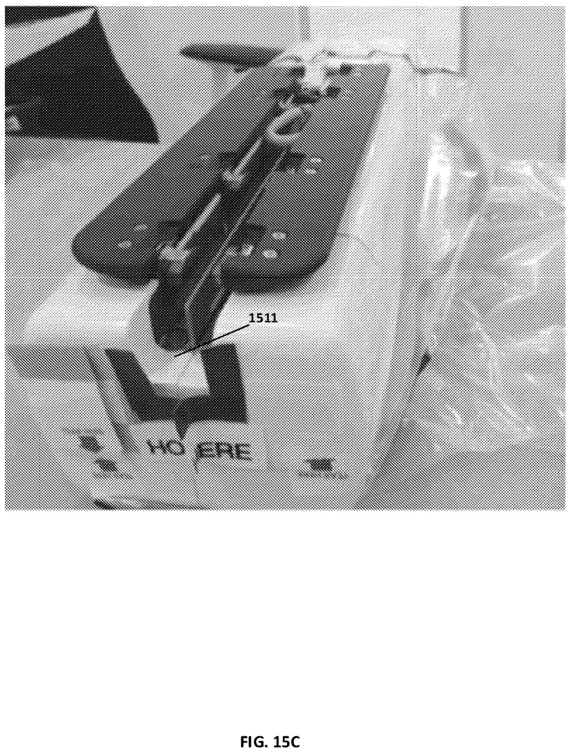

Figures 15A-C are images of a drape comprising an opening shaped and sized to expose an access port and an exit aperture of a motor unit when dressed on a motor unit, according to some embodiments. In some embodiments, as shown for example in figure 15A, drape 1501 defines an opening having a stepped profile. Optionally, a first step 1503 is shaped, sized and positioned to conform to an access port 1505 of motor unit 1507, as shown for example in figure 15B; and a second step 1509 is shaped, sized and positioned for exposing an exit aperture 1511 of the motor unit, e.g. as shown in figure 15C, optionally located on a front face of motor unit 1507.

In some embodiments, one or more attachment means 1513 such as adhesives (e.g. stickers, pull-off tabs), magnets, hook and loop fasteners and/or other attachment means are positioned at one or more locations along the borders of the opening in the drape, to attach the drape to the motor unit.

In some embodiments, a marker 1515, for example in the form of a line of tape, a colored line and/or other type of marker extends to differentiate a sterile portion of the drape (e.g. when deployed onto the motor unit) from a non-sterile portion. In some embodiments, access port borders are located within the borders defined by the marker.

Figures 17A-D schematically illustrate a drape for covering a surgical device and at least a portion of a base on which the surgical device is mounted, according to some embodiments.

In some embodiments, drape 1701 (see outline in Figure 17D) is shaped and/or sized to cover a surgical device, e.g. a motor unit 1703 (see Figure 17A), and at least a portion of a base 1705 (see Figure 17A) on which the motor unit is seated or mounted. In some embodiments, the base is movable, for example a rolling cart.

In some embodiments, drape 1701 comprises a first opening 1702 (which may also be referred to as "accessible opening") for exposing an access port 1707 of the motor unit, and a second opening 1713 (which may also be referred to as "expansion opening") which facilitates covering base 1705, which, in some embodiments, may be of larger dimensions (e.g. larger width, length and/or height) relative to the motor unit. In an example, a width 1709 of base 1705 is about 25%, 50%, 75%, 100%, 125% larger than a width 1711 of motor unit 1703.

A potential advantage of covering the base with the same drape used for covering the motor unit may include facilitating use, reducing the number of actions required to be performed by the physician/nurse in preparation for surgery, thereby reducing time spent on preparations such as maintenance of sterility using the drape.

In some embodiments, a tear-off piece and/or other removable or movable drape portion defines second opening 1713 such that upon its removal, deploying of drape 1701 onto the base 1705 (e.g. from the top downwards) is facilitated.

In some embodiments, an upper drape sheet and/or other drape portion or even a sterile sheet are placed on top of opening 1713 after drape 1701 is deployed. Optionally, as shown for example in figure 17B, one or more attachment means 1715 such as adhesives (e.g. stickers, optionally double sided); clips; magnets and/or other attachment means are positioned on drape 1701 for attaching to an upper drape or other portion covering opening 1713.

In some embodiments, as shown for example in figure 17C, drape 1701 comprises a second layer 1717 which can be moved, unrolled and/or otherwise positioned to cover opening 1713.

Figure 17D shows an example of a drape layout including a cut 1719 which allows for pulling drape portions configured on its sides apart from each other, so as to form opening 1713. In some embodiments, cut 1719 extends along a segment of the drape long axis. In some embodiments, opening 1702 for allowing access to the access port is configured opposite cut 1719, e.g. along the drape long axis. In some embodiments, a fixation 1721 such as a deformable strip is positioned intermediate cut 1719 and opening 1702.

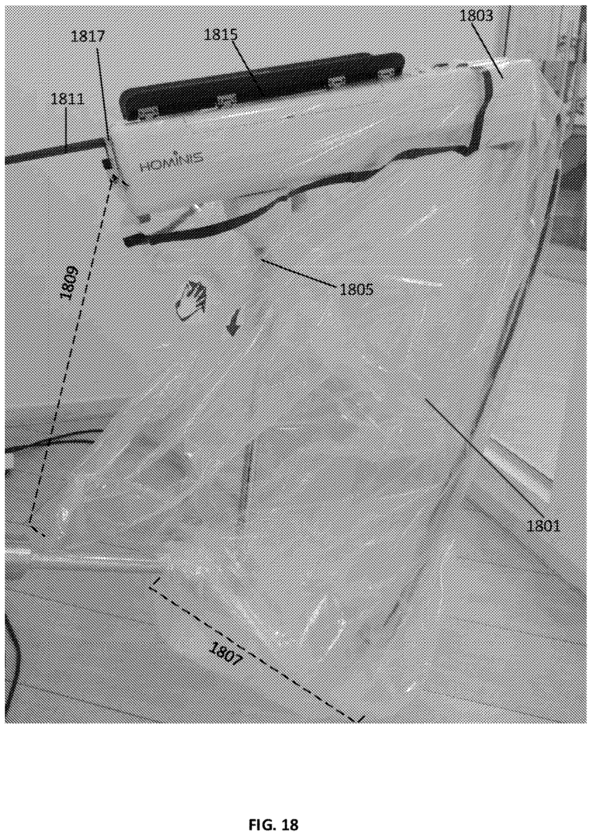

**Figure 18** is an image of drape covering a motor unit while exposing an access port and an exit aperture of the motor unit, according to some embodiments.

In the exemplary assembly shown in figure 18, a drape 1801 is shown to cover, in part, a motor unit 1803 which is mounted on a stand (or base) 1805. In some embodiments, drape 1801 is sized to be large enough to cover stand 1805 along the height of the stand. Optionally, the drape is large enough to extend from the motor unit position to the ground on which the stand is positioned.

In some embodiments, drape 1801 is a sleeve, comprising an elongated hollow structure, optionally tubular.

In some embodiments, an open end 1807 of the deployed drape is directed towards the grounds. Optionally, free ends of the open end may be tied or fastened together.

In some embodiments, a partially closed end 1809 of the deployed drape faces a distal (front) direction. Optionally, this direction corresponds with a direction in which the one or more surgical arms 1811 extend from the motor unit. In some embodiments, in a non-deployed state, the partially closed end 1809 is configured opposite the open end 1807, and upon dressing the drape on the device, a portion of the drape is rotated, moving the partially closed end to face a direction which is substantially perpendicular to a direction which the open end faces. In some embodiments, this rotation is a result of aligning an accessible opening of the drape with the access port 1815 and/or exit aperture 1817 of the motor unit 1803.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

## Claims

1. A two-portion drape for a surgical device, comprising:
I. a first drape portion (1401) formed of a thin sheet of flexible material defining an elongated hollow sleeve, the first drape portion (1401) comprising:
an open end (1451) on a first side of the sleeve;
a partially closed end (1453) on a second, opposing side of the sleeve;
an elongated sleeve body extending between the open end (1451) and the partially closed end (1453); and
an accessible opening (1407) formed as a cut-out which extends from the partially closed end (1453) to an adjacent portion of the sleeve body and exposes at least a part of the surgical device when said first drape is placed thereon;
II. a second drape portion configured for deployment over said first drape portion (1401), said second drape portion formed of a thin sheet of flexible material, said second drape portion shaped and sized to cover said accessible opening (1407) at least in part.

2. The drape according to claim 1, wherein said body of said first drape portion (1401) comprises at least one fixation element (1415) embedded or mounted onto said body and located between said accessible opening (1407) and said open end (1451), said fixation element (1415) comprising an elongated deformable element (713).

3. The drape according to claim 1 or claim 2, wherein said body of said first drape portion (1401) is closed along the long edges of body.

4. The drape according to any of claims 1-3, wherein said accessible opening (1407) is rectangular.

5. The drape according to any of claims 1-4, wherein said second drape portion comprises a handling portion including valley folds (415) each sized for insertion of at least a part of a user's hand.

6. The drape according to any of claims 1-5, formed of continuous or welded plastic or nylon sheets, including one or more of: thermoplastic polyurethane, ultra-high molecular weight polyethylene, low density polyethylene, and high density polyethylene.

7. The drape according to any of claims 1-6, wherein said drape is at least partially transparent to allow visibility therethrough of at least a portion of a surgical device covered by said drape.

8. The drape according to any of claims 1-7, wherein said first drape portion (1401) comprises a marker (1515) which extends at least around a perimeter of said accessible opening (1407).

9. The drape according to any of claims 1-8, wherein said first drape portion (1401) comprises a pocket or fold (903) in which said second drape portion is stored and can be pulled away from or inserted within.

10. The drape according to claim 1, wherein said first drape portion (1401) and said second drape portion are unitarily formed.

11. The drape according to claim 1, wherein said first drape portion (1401) and said second drape portion are formed as separate sheets.

12. The drape according to any of claims 1-11, wherein one or both of said first drape portion (1401) and said second drape portion comprise attachment means (709) including one or more of: adhesives, hook and loop fasteners, velcro, magnets.

13. The drape according to any of claims 1-12, wherein one or both of said first drape portion (1401) and said second drape portion comprise one or more markings (1031) for indicating a direction of dressing the drape portion over a surgical device.

14. The drape according to any of claims 1-13, wherein said first drape portion (1401) comprises a detachable portion which when detached or moved forms said accessible opening.

15. A method for sterile covering of a surgical device including an access port and a surgical arm, the method comprising:
deploying an elongated hollow sleeve drape including an opening to cover the surgical device while exposing the access port via the opening;
affixing the elongated hollow sleeve drape onto the surgical device such that said opening is maintained in alignment with said access port, providing consistent access to said access port;
attaching said surgical arm to said surgical device via said access port, and
covering said access port with a second drape, said second drape being a thin sheet of flexible material, or drape portion, said drape portion being a thin sheet of flexible material, such that only said surgical arm protrudes therefrom.

## Patentansprüche

1. Abdecktuch mit zwei Abschnitten für eine chirurgische Vorrichtung, Folgendes umfassend:
I. einen ersten Abdecktuchabschnitt (1401), der aus einer dünnen Folie aus flexiblem Material gebildet ist und eine längliche hohle Hülse definiert, wobei der erste Abdecktuchabschnitt (1401) Folgendes umfasst:
ein offenes Ende (1451) auf einer ersten Seite der Hülse;
ein teilweise geschlossenes Ende (1453) auf einer zweiten, gegenüberliegenden Seite der Hülse;
einen länglichen Hülsenkörper, der sich zwischen dem offenen Ende (1451) und dem teilweise geschlossenen Ende (1453) erstreckt; und
eine zugängliche Öffnung (1407), die als eine Aussparung gebildet ist, die sich von dem teilweise geschlossenen Ende (1453) zu einem benachbarten Abschnitt des Hülsenkörpers erstreckt und mindestens einen Teil der chirurgischen Vorrichtung freilegt, wenn das erste Abdecktuch darauf platziert ist;
II. einen zweiten Abdecktuchabschnitt, der konfiguriert ist, um über dem ersten Abdecktuchabschnitt (1401) entfaltet zu werden, wobei der zweite Abdecktuchabschnitt aus einer dünnen Folie aus flexiblem Material gebildet ist, wobei der zweite Abdecktuchabschnitt geformt und bemessen ist, um die zugängliche Öffnung (1407) mindestens teilweise abzudecken.

2. Abdecktuch nach Anspruch 1, wobei der Körper des ersten Abdecktuchabschnitts (1401) mindestens ein Fixierungselement (1415) umfasst, das in dem Körper eingebettet oder an diesem montiert ist und sich zwischen der zugänglichen Öffnung (1407) und dem offenen Ende (1451) befindet, wobei das Fixierungselement (1415) ein längliches verformbares Element (713) umfasst.

3. Abdecktuch nach Anspruch 1 oder Anspruch 2, wobei der Körper des ersten Abdecktuchabschnitts (1401) entlang der langen Kanten des Körpers geschlossen ist.

4. Abdecktuch nach einem der Ansprüche 1-3, wobei die zugängliche Öffnung (1407) rechteckig ist.

5. Abdecktuch nach einem der Ansprüche 1-4, wobei der zweite Abdecktuchabschnitt einen Handhabungsabschnitt umfasst, der Talfalten (415) beinhaltet, die jeweils zum Einführen von mindestens einem Teil der Hand eines Benutzers bemessen sind.

6. Abdecktuch nach einem der Ansprüche 1-5, das aus durchgehenden oder geschweißten Kunststoff- oder Nylonfolien gebildet ist, beinhaltend eines oder mehrere von Folgendem: thermoplastisches Polyurethan, ultrahochmolekulargewichtiges Polyethylen, Polyethylen niedriger Dichte und Polyethylen hoher Dichte.

7. Abdecktuch nach einem der Ansprüche 1-6, wobei das Abdecktuch mindestens teilweise transparent ist, um eine Sichtbarkeit mindestens eines Abschnitts einer chirurgischen Vorrichtung, die durch das Abdecktuch abgedeckt ist, durch dieses zu ermöglichen.

8. Abdecktuch nach einem der Ansprüche 1-7, wobei der erste Abdecktuchabschnitt (1401) eine Markierung (1515) umfasst, die sich mindestens um einen Umfang der zugänglichen Öffnung (1407) erstreckt.

9. Abdecktuch nach einem der Ansprüche 1-8, wobei der erste Abdecktuchabschnitt (1401) eine Tasche oder Falte (903) umfasst, in der der zweite Abdecktuchabschnitt gelagert ist und weggezogen oder darin eingeführt werden kann.

10. Abdecktuch nach Anspruch 1, wobei der erste Abdecktuchabschnitt (1401) und der zweite Abdecktuchabschnitt einstückig gebildet sind.

11. Abdecktuch nach Anspruch 1, wobei der erste Abdecktuchabschnitt (1401) und der zweite Abdecktuchabschnitt als getrennte Folien gebildet sind.

12. Abdecktuch nach einem der Ansprüche 1-11, wobei einer oder beide des ersten Abdecktuchabschnitts (1401) und des zweiten Abdecktuchabschnitts Befestigungsmittel (709) umfassen, die eines oder mehrere von Folgendem beinhalten: Klebstoffe, Klettverschlüsse, Velcro-Klett, Magnete.

13. Abdecktuch nach einem der Ansprüche 1-12, wobei einer oder beide des ersten Abdecktuchabschnitts (1401) und des zweiten Abdecktuchabschnitts eine oder mehrere Markierungen (1031) zum Angeben einer Richtung zum Anlegen des Abdecktuchabschnitts über einer chirurgischen Vorrichtung umfassen.

14. Abdecktuch nach einem der Ansprüche 1-13, wobei der erste Abdecktuchabschnitt (1401) einen lösbaren Abschnitt umfasst, der, wenn er abgelöst oder bewegt wird, die zugängliche Öffnung bildet.

15. Verfahren zum sterilen Abdecken einer chirurgischen Vorrichtung beinhaltend eine Zugangsanschluss und einem chirurgischen Arm, wobei das Verfahren Folgendes umfasst:
Entfalten eines länglichen hohlen Hülsenabdecktuchs beinhaltend eine Öffnung, um die chirurgische Vorrichtung abzudecken, während der Zugangsanschluss über die Öffnung freigelegt wird;
Fixieren des länglichen hohlen Hülsenabdecktuchs auf der chirurgischen Vorrichtung, sodass die Öffnung in Ausrichtung mit dem Zugangsanschluss gehalten wird, wodurch ein ständiger Zugang zu dem Zugangsanschluss bereitgestellt wird;
Befestigen des chirurgischen Arms an der chirurgischen Vorrichtung über den Zugangsanschluss und
Abdecken des Zugangsanschlusses mit einem zweiten Abdecktuch, wobei das zweite Abdecktuch eine dünne Folie aus flexiblem Material oder ein Abdecktuchabschnitt ist, wobei der Abdecktuchabschnitt eine dünne Folie aus flexiblem Material ist, sodass nur der chirurgische Arm davon vorsteht.

## Revendications

1. Champ en deux parties pour un dispositif chirurgical, comprenant :
I. une première partie de champ (1401) formée d'une fine feuille de matériau flexible définissant un manchon creux allongé, la première partie de champ (1401) comprenant :
une extrémité ouverte (1451) sur un premier côté du manchon ;
une extrémité partiellement fermée (1453) sur un second côté opposé du manchon ;
un corps de manchon allongé se prolongeant entre l'extrémité ouverte (1451) et l'extrémité partiellement fermée (1453) ; et
une ouverture accessible (1407) formée comme une découpe qui se prolonge de l'extrémité partiellement fermée (1453) à une partie adjacente du corps de manchon et expose au moins une partie du dispositif chirurgical lorsque ledit premier champ est placé dessus ;
II. une seconde partie de champ configurée pour être déployée sur ladite première partie de champ (1401), ladite seconde partie de champ étant formée d'une fine feuille de matériau flexible, ladite seconde partie de champ étant formée et dimensionnée pour couvrir ladite ouverture accessible (1407) au moins en partie.

2. Champ selon la revendication 1, dans lequel ledit corps de ladite première partie de champ (1401) comprend au moins un élément de fixation (1415) intégré ou monté sur ledit corps et situé entre ladite ouverture accessible (1407) et ladite extrémité ouverte (1451), ledit élément de fixation (1415) comprenant un élément déformable allongé (713).

3. Champ selon la revendication 1 ou la revendication 2, dans lequel ledit corps de ladite première partie de champ (1401) est fermé le long des bords longs du corps.

4. Champ selon l'une quelconque des revendications 1 à 3, dans lequel ladite ouverture accessible (1407) est rectangulaire.

5. Champ selon l'une quelconque des revendications 1 à 4, dans lequel ladite seconde partie de champ comprend une partie de manipulation comportant des plis de vallée (415), dimensionnés chacun pour l'insertion d'au moins une partie de la main d'un utilisateur.

6. Champ selon l'une quelconque des revendications 1 à 5, formé de feuilles de plastique ou de nylon continues ou soudées, comportant l'un ou plusieurs : de polyuréthane thermoplastique, de polyéthylène à poids moléculaire ultra élevé, de polyéthylène basse densité et de polyéthylène haute densité.

7. Champ selon l'une quelconque des revendications 1 à 6, dans lequel ledit champ est au moins partiellement transparent pour permettre la visibilité à travers celui-ci d'au moins une partie d'un dispositif chirurgical recouvert par ledit champ.

8. Champ selon l'une quelconque des revendications 1 à 7, dans lequel ladite première partie de champ (1401) comprend un marqueur (1515) qui se prolonge au moins autour d'un périmètre de ladite ouverture accessible (1407).

9. Champ selon l'une quelconque des revendications 1 à 8, dans lequel ladite première partie de champ (1401) comprend une poche ou un pli (903) dans lequel ladite seconde partie de champ est stockée et peut être retirée ou insérée à l'intérieur.

10. Champ selon la revendication 1, dans lequel ladite première partie de champ (1401) et ladite seconde partie de champ sont formées de manière unitaire.

11. Champ selon la revendication 1, dans lequel ladite première partie de champ (1401) et ladite seconde partie de champ sont formées sous forme de feuilles séparées.

12. Champ selon l'une quelconque des revendications 1 à 11, dans lequel l'une ou les deux de ladite première partie de champ (1401) et de ladite seconde partie de champ comprennent un moyen d'attache (709) comportant l'un ou plusieurs : d'adhésifs, d'attaches à crochets et boucles, de velcro, d'aimants.

13. Champ selon l'une quelconque des revendications 1 à 12, dans lequel l'une ou les deux de ladite première partie de champ (1401) et de ladite seconde partie de champ comprennent un ou plusieurs marquages (1031) pour indiquer une direction d'habillage de la partie de champ sur un dispositif chirurgical.

14. Champ selon l'une quelconque des revendications 1 à 13, dans lequel ladite première partie de champ (1401) comprend une partie détachable qui, lorsqu'elle est détachée ou déplacée, forme ladite ouverture accessible.

15. Procédé de recouvrement stérile d'un dispositif chirurgical comportant un orifice d'accès et un bras chirurgical, le procédé comprenant :
le déploiement d'un champ à manchon creux allongé comportant une ouverture pour couvrir le dispositif chirurgical tout en exposant l'orifice d'accès via l'ouverture ;
la fixation du champ à manchon creux allongé sur le dispositif chirurgical de telle sorte que ladite ouverture soit maintenue en alignement avec ledit orifice d'accès, fournissant un accès constant audit orifice d'accès ;
l'attachement dudit bras chirurgical audit dispositif chirurgical via ledit orifice d'accès, et
le recouvrement dudit orifice d'accès avec un second champ, ledit second champ étant une fine feuille de matériau flexible, ou une partie de champ, ladite partie de champ étant une fine feuille de matériau flexible, de telle sorte que seul ledit bras chirurgical en dépasse.
